# EUROPÄISCHE PATENTSCHRIFT

(11) **EP 1 987 128 B1**
(45) Veröffentlichungstag und Bekanntmachung des Hinweises auf die Patenterteilung: **13.10.2010**
(21) Anmeldenummer: 07712281.0
(22) Anmeldetag: 21.02.2007
(51) Int. Cl.: C12M 1/107

(54) **BIOREAKTOR ZUR METHANISIERUNG VON BIOMASSE MIT HOHEM FESTSTOFFANTEIL**
BIOREACTOR FOR METHANIZATION OF BIOMASS HAVING A HIGH SOLIDS FRACTION
BIORÉACTEUR POUR LA MÉTHANISATION DE BIOMASSE À TENEUR EN SOLIDES ÉLEVÉE

(30) Priorität: 21.02.2006 DE 202006002757 U
(43) Veröffentlichungstag der Anmeldung: 05.11.2008
(73) Patentinhaber: Bekon Energy Technologies GmbH & CO. KG, 85774 Unterföhring (DE)
(72) Erfinder: LUTZ, Peter, 81927 München (DE)
(74) Vertreter: Winter, Brandl, Fürniss, Hübner, Röss, Kaiser, Polte - Partnerschaft
(86) Internationale Anmeldenummer: PCT/EP2007/051686
(87) Internationale Veröffentlichungsnummer: WO 2007/096392

(56) Entgegenhaltungen:
- DE-A1- 3 719 564
- DE-A1- 4 444 462
- DE-U1- 20 121 701
- FR-A1- 2 502 174

## Beschreibung

Die Erfindung betrifft einen Bioreaktor zur Methanisierung von Biomasse mit hohem Feststoffanteil nach dem Oberbegriff des Anspruch 1.

Der Begriff "Biomasse mit hohem Feststoffanteil" ist als Gegensatz zu flüssiger, pumpbarer Biomasse, wie sie in Nassgärverfahren eingesetzt wird, zu verstehen. "Biomasse mit hohem Feststoffanteil" ist daher als nichtpumpbare Biomasse zu verstehen.

Ein Bioreaktor nach dem Oberbegriff des Anspruchs 1 ist aus der EP 1 301 583 B1 bekannt, auf deren Offenbarung zur Vermeidung unnötiger Wiederholung vollinhaltlich Bezug genommen und deren Offenbarungsgehalt ausdrücklich in die vorliegende Anmeldung einbezogen wird.

Dieser bekannten Bioreaktor umfasst einen Faulbehälter nach Art einer Fertiggarage, der mittels einer Klappe gasdicht verschlossen wird. Die Beladung mit unvergorener Biomasse und die Entladung der vergorenen Restbiomasse kann mittels eines Radladers erfolgen. Da sich die Biomasse während des Vergärungsprozesses setzt, drückt die Biomasse gegen die Klappe. Die Klappe muss deshalb entsprechend massiv ausgeführt sein und muss andererseits zur ausreichenden Abdichtung des Faulbehälters auch präzise schließen. Eine solche zugleich feste und präzise bewegliche Klappe ist teuer. Zugleich besteht die Gefahr, dass die Klappe bei hoher Beladung mit Biomasse nicht mehr präzise schließt und abdichtet. Um dieses Problem zu lösen schlägt die DE 202005014176.3 ein Rückhaltesystem hinter der Klappe vor, das verhindert, dass die sich setzende Biomasse gegen die Klappe drückt.

In DE 102 57 849 A1 ist ein Garagen-Fermenter beschrieben, bei dem durch einen Siebboden die Biomasse von unten mit Biogas belüftet wird. Dieser Siebboden fungiert somit auch als Drainageeinrichtung für das Perkolat, welches wie bei uns auch umgepumpt wird. Eine Regelung des Füllstands wird jedoch nicht erwähnt.

Die DE 101 29 718 A1 beschreibt einen vertikalen Fermenter zur Methanisierung von häuslichem Biomüll mit kontinuierlicher Beschickung und Entnahme mittels Schneckenförderer durch separate Öffnungen. Das Perkolat wird über einen Siebboden abgetrennt und umgepumpt, dessen Füllstand aber nicht geregelt.

Die DE 44 44 462 A1 betrifft ebenfalls einen vertikalen Fermenter, bei dem die Biomasse oben diskontinuierlich und gasdicht eingefüllt wird, während der Fermentation nach unten absinkt und dort diskontinuierlich und gasdicht entnommen wird. Es wird eine gesteuerte Sickerwasserkreislaufführung verwendet, um die oben gebildeten organischen. Säuren in den unteren Bereich des Reaktors zu transportieren, wo das Vergären stattfindet. Der Feststoffanteil des biogenen Materials wird mit 30% bis 55% angebeben.

In der DE 201 21 701 U1 wird ein zweistufiges System mit zwei Reaktoren beschrieben. Im ersten Reaktor wird in einem Nassverfahren die Biomasse schwimmend in einer Behandlungsflüssigkeit, welche anaerobe Bakterien enthält, gehalten und so von einer Zugabeöffnung zu einer Entnahmeöffnung transportiert. Die Behandlungsflüssigkeit wird durch Kreislaufführung auf die Biomasse geregnet. Die Behandlungsflüssigkeit wird in einen zweiten Reaktor übertragen und dort vergoren. Es entsteht jedoch in beiden Reaktoren Biogas. Als Alternative zum Nassverfahren kann im ersten Reaktor auch Biomasse mit hohem Feststoffanteil behandelt werden. In diesem Fall wird Biogas hauptsächlich im zweiten Reaktor erzeugt. Eine Regelung des Füllstandes für die Behandlungsflüssigkeit wird nicht erwähnt.

Die EP 0 803 568 A1 beschreibt eine Biogasanlage zum Vergären von Biomasse mit einem Feststoffanteil von etwa 25% Trockensubstanz. Die Anlage besteht aus mehreren Reaktoren die untereinander so verbunden sind, dass aus den Reaktoren entnommenes Material gezielt anderen Reaktoren zugeführt werden kann, um die Prozessführung in jedem Reaktor individuell und optimal zu gestalten.

Die DE 37 19 564 A1 offenbart einen Bioreaktor nach dem Oberbegriff des Anspruchs 1.

Obwohl die Gasausbeute aus der Biomasse und die notwendige Verweildauer der Biomasse in vielen Fällen ausreichend ist, wäre eine höhere Gasausbeute bzw. eine geringere Verweilzeit der Biomasse in dem Faulbehälter wünschenswert.

Es ist daher Aufgabe der vorliegenden Erfindung, den aus der DE 3719564 A1 bekannten Bioreaktor dahingehend zu verbessern, dass die Gasausbeute erhöht und die notwendige Verweildauer der Biomasse in dem Faulbehälter verringert wird.

Zur Lösung dieser Aufgabe ist ein Bioreaktor nach dem Oberbegriff des Anspruchs 1 durch dessen kennzeichnende Merkmale weitergebildet.

Beim Vergären von trockener, d.h. nicht pumpfähiger Biomasse, entstehen aufgrund der in der Biomasse enthaltenen Feuchtigkeit Sickersäfte, sogenanntes Perkolat, das über eine Drainageeinrichtung abgezogen und ggfs. wieder von oben auf die zu vergärende Biomasse zurückgeführt wird. Es hat sich nun herausgestellt, dass die Biogasausbeute wesentlich erhöht wird - im Bereich zwischen 10% und 40% -, wenn das entstehende Perkolat nicht sofort über die Drainageeinrichtung abgezogen, sondern in den Faulbehälter bis zu einem bestimmten Pegel aufgestaut wird. Dies wird vorrichtungstechnisch dadurch erreicht, dass der Faulbehälter flüssigkeitsdicht ausgelegt ist, d.h. auch die Klappe zum Be- und Entladen des Faulbehälters muss flüssigkeitsdicht ausgeführt werden und auch entsprechend stabil ausgestaltet sein, um dem entstehenden Flüssigkeitsdruck Stand zu halten. Durch die Verbindung der vorhandenen Perkolat-Drainageeinrichtung mit einer Perkolat-S,teuereinrichtung ist es möglich, den Flüssigkeitspegel des Perkolats in der vergärenden Biomasse so einzustellen und zu regeln, dass die Biogaserzeugungsrate oder die Biogasausbeute maximal wird.

Gemäß einer bevorzugten Ausführungsform der Erfindung nach Anspruch 2 umfasst die Perkolat-Steuereinrichtung eine Perkolatspeicher der über eine Perkolat-Ableitung mit der Perkolat-Drainageeinrichtung verbunden ist. In der Perkolat-Ableitung ist ein Ventil angeordnet, mittels dem das sich in dem Faulbehälter bildende Perkolat zu der gewünschten Höhe aufgestaut werden kann. Die Höhe des Perkolatpegels wird hierbei so eingestellt, dass die Gasausbeute maximal wird. Die Ableitung des Perkolats in den Perkolatspeicher erfolgt mittels Schwerkraft.

Gemäß einer vorteilhaften Ausgestaltung der Erfindung nach Anspruch 3 ist in der Perkolat-Ableitung eine Perkolat-Pumpe angeordnet, um die Ableitung des Perkolats in den Perkolatspeicher von der Schwerkraft unabhängig zu ermöglichen. Hierbei kann die Perkolat-Pumpe auch zugleich die Funktion des Ventils übernehmen.

Gemäß einer vorteilhaften Ausgestaltung der Erfindung nach Anspruch 4 handelt es sich bei dem Füllstandssensor um einen einfachen Drucksensor, der in der Perkolat-Ableitung im Bereich des Bodens des Faulbehälters angeordnet ist. Derartige Drucksensoren sind sehr preisgünstig und gegenüber dem chemisch agressiven Perkolat widerstandsfähig.

Gemäß einer weiteren bevorzugten Ausführungsform der Erfindung nach Anspruch 5 umfasst die Perkolat-Steuereinrichtung auch eine Perkolat-Rückleitung, um in an sich bekannter Weise Perkolat aus dem Perkolatspeicher wieder in dem Faulbehälter zurückzuführen.

Gemäß einer weiteren bevorzugten Ausführungsform der Erfindung nach Anspruch 8 sind in den Perkolatspeicher Füllkörper eingebracht, in denen sich Biogas erzeugende Mikroorganismen besonders gut anlagern können. Auf einfache Weise wird dies gemäß Anspruch 9 dadurch erreicht, dass in den Perkolatspeicher ein Festbett aus Biomasse mit hohem Feststoffanteil eingegeben wird. Auf diese Weise dient auch der Perkolatspeicher selbst als Bioreaktor und es kann aus dem Perkolatspeicher Biogas abgezogen werden.

Gemäß einer weiteren bevorzugen Ausgestaltung - Anspruch 10 - lassen sich eine Mehrzahl der erfindungsgemäßen Bioreaktoren zusammenschalten, um eine Biogaserzeugungsvorrichtung bereitzustellen. Die Mehrzahl der Bioreaktoren umfasst dann einen gemeinsamen Perkolatspeicher.

Gemäß einer bevorzugten Ausführungsform der Erfindung nach Anspruch 11 sind hierbei die einzelnen Bioreaktoren zusätzlich über Perkolat-Verbindungsleitungen untereinander verbunden. Hierdurch läßt sich beispielsweise der gleiche Flüssigkeitspegel in den einzelnen Bioreaktoren einstellen.

Weitere Einzelheiten, Merkmale und Vorteile der Erfindung ergeben sich aus der nachfolgenden Beschreibung der beispielhaften Ausführungsformen der Erfindung anhand der Zeichnungen.

Es zeigt:
Fig. 1 schematisch eine perspektivische Darstellung des erfindungsgemäßen Bioreaktors;
Fig. 2 eine Schnittdarstellung des Bioreaktors nach Fig. 1 quer zur Be- und Entladerichtung;
Fig. 3 eine schematische Darstellung der Bodenplatte des Bioreaktors aus Fig. 1 bzw. 2;
Fig. 4 eine schematische Schnittdarstellung des Bioreaktors nach den Figuren 1 bis 3; und
Fig. 5 eine schematische Darstellung einer beispielhaften Ausführungsform einer Biogaserzeugungsvorrichtung mit einer Mehrzahl von erfindungsgemäßen Bioreaktoren.

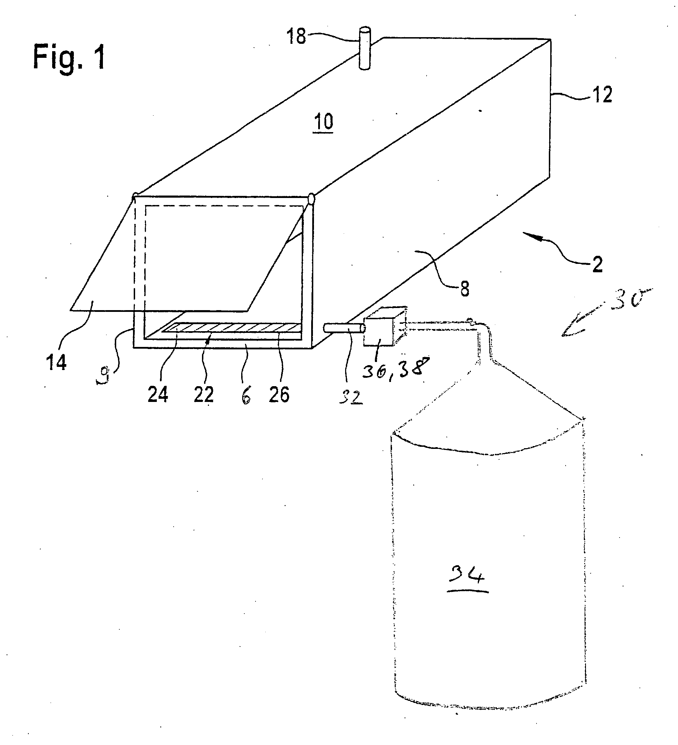

Die Figuren 1 bis 4 zeigen eine beispielhafte Ausführungsform Bioreaktor bzw. Biogasreaktor 1 gemäß der vorliegenden Erfindung. Der Bioreaktor umfasst einen quaderförmigen Faulbehälter 2, der mit Biomasse 4 gefüllt ist. Der Faulbehälter 2 besteht nach Art einer Fertiggarage aus Stahlbeton und umfasst sechs plane Wandelemente, nämlich eine Bodenplatte 6, zwei Seitenwände 8 und 9, eine Deckplatte 10, eine Rückwand 12 und eine offene Vorderseite 13, die durch eine gasdichte Klappe 14 verschließbar ist.

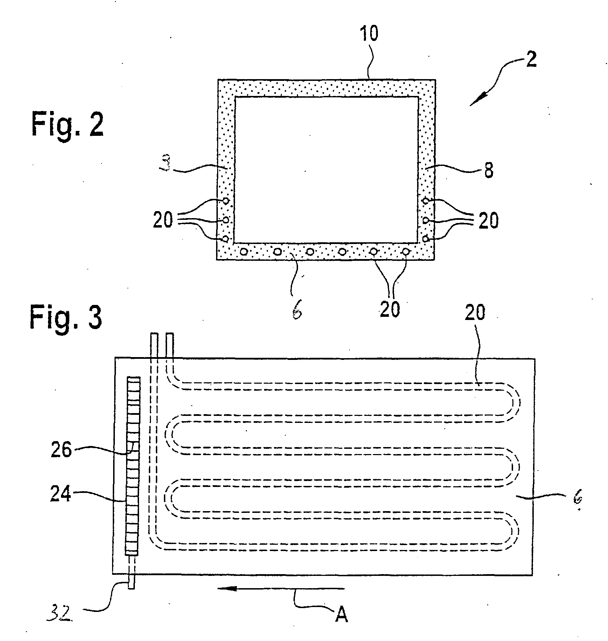

Die Klappe 14 ist mittels einer Hydraulik 16 betätigbar. Bei offener Klappe 14 lässt sich der Faulbehälter 2 auf einfache Weise befüllen bzw. die Restbiomasse daraus entfernen. Über eine Biogasableitung 18 wird das in dem Faulbehälter 2 erzeugte Biogas abgeführt. In der Bodenplatte 6 des Faulbehälters 2 und teilweise auch in den Seitenwänden 8 und 9 kann eine Heizeinrichtung 20 - siehe Fig. 3 - nach Art einer Fußbodenheizung vorgesehen sein, mittels der die in dem Faulbehälter 2 befindliche Biomasse 4 entsprechend temperiert werden kann. Hinsichtlich Details wird hierzu auf die entsprechenden Ausführungen in der EP 1 301 583 B1 verwiesen.

Ebenfalls in der Bodenplatte 6 integriert ist eine Sickersaft-Drainageeinrichtung 22, die durch eine leichte Neigung der Bodenplatte 6 in Richtung Klappe 14 und durch eine im vorderen Bereich des Faulbehälters hinter der Klappe 14 angeordneten Rinne 24 realisiert ist. Die Rinne 24 verläuft quer zur Längs- oder Be- und Entladerichtung des Faulbehälters 2 und wird durch ein Loch- oder Schlitzblech 26 abgedeckt. In der beispielhaften Ausführungsform ist lediglich eine Rinne 24 dargestellt. Alternativ können mehrere solcher Rinnen vorgesehen werden, die ebenfalls quer oder in Längsrichtung angeordnet sein können.

Die Höhe des Perkolatpegels 28 wird mittels einer Perkolat-Steuereinrichtung 30 geregelt bzw. gesteuert. Die Perkolat-Steuereinrichtung 30 umfasst eine Sickersaft- bzw. Perkolat-Ableitung 32 mittels der das sich in der Rinne 24 sammelnde Perkolat in einen Perkolatspeicher 34 abgeleitet wird. Die Perkolat-Ableitung 32 ist mittels eines Ventils 36 abschließbar, so dass das sich in dem Faulbehälter 2 sammelnde Perkolat bis zu einem gewünschten Pegel 28 aufgestaut werden kann. In der Perkolat-Ableitung 32 ist eine Perkolat-Pumpe 38 angeordnet, um das Perkolat unabhängig von der Schwerkraft in den Perkolatspeicher 34 befördern zu können.

Über einen Füllstandssensor in Form eines Drucksensors 40 wird die Höhe des Perkolatpegels 28 in dem Faulbehälter 2 ermittelt. Der Drucksensor 40 ist in der Perkolat-Ableitung 32 vor dem Ventil 36 angeordnet. Der Drucksensor 40, das Ventil 36 und die Perkolat-Pumpe 38 sind über Steuerleitungen 42 mit einer Steuereinheit 44 verbunden. Über zusätzliche Steuerleitungen 46 können der Steuereinheit 44 weitere Prozessparameter, wie z. B. die Gasproduktionsrate, der Gasdruck im Inneren des Faulbehälters etc. zugeführt werden. Auf diese Weise kann mittels der Steuereinheit 44 der Perkolatpegel 28 so eingestellt werden, dass die Gasproduktionsrate auf einem maximalen Wert gehalten wird.

In Fig. 1, die nur eine schematische Darstellung einer Ausführungsform der Erfindung zeigt, ist der Perkolat-Speicher 34 unter dem Faulbehälter angeordnet. Da in der Perkolat-Ableitung 32 eine Perkolat-Pumpe 38 vorgesehen ist, kann der Perkolat-Speicher 34 auch auf gleicher Höhe mit dem Faulbehälter 2 angeordnet werden.

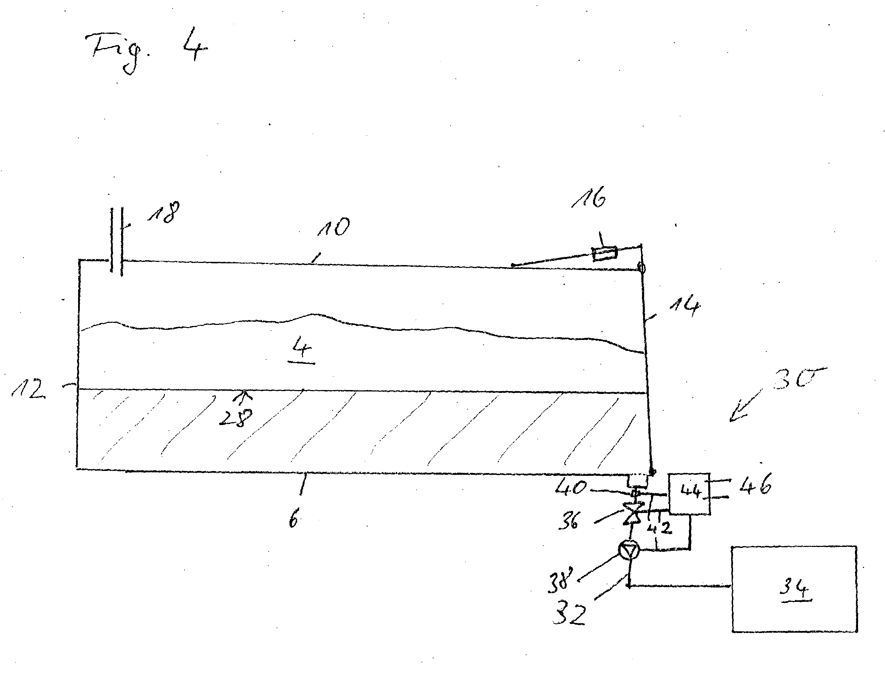

In Fig. 4 ist das Ventil 36 in Abflussrichtung vor der Perkolat-Pumpe 38 angeordnet. Alternativ kann das Ventil 36 auch nach der Perkolat-Pumpe 38 angeordnet werden. In diesem Fall kann auch der Drucksensor 40 im Pumpensumpf der Perkolat-Pumpe 38 angeordnet werden. Bei entsprechender Ausgestaltung der Perkolat-Pumpe 38 kann diese auch die Funktion des Ventils 36 übernehmen, d. h. im Stillstand schließt die Perkolat-Pumpe 36 die Perkolat-Ableitung 32 ab, so dass das Perkolat im Faulbehälter aufgestaut werden kann.

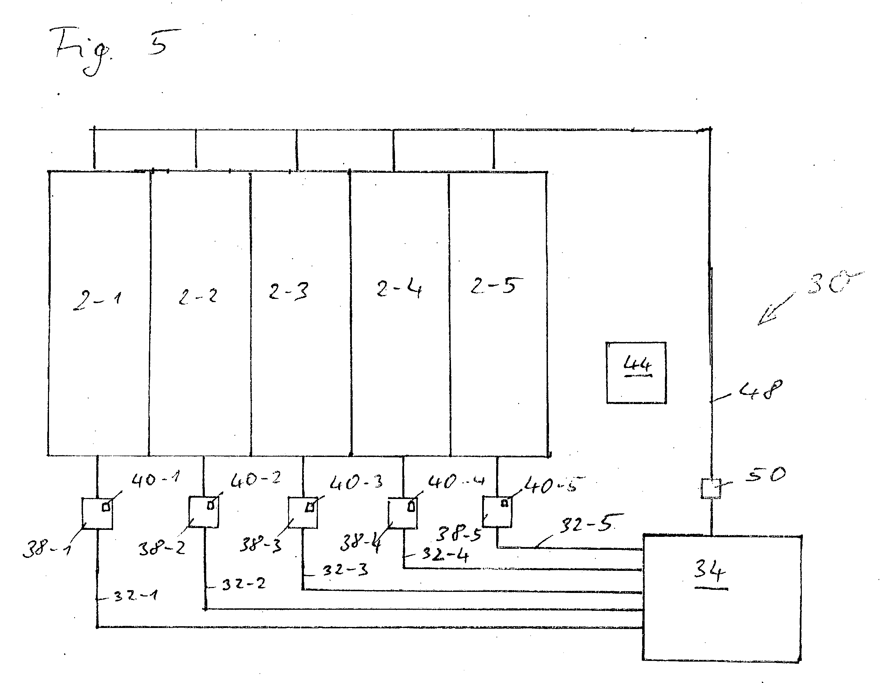

Fig. 5 zeigt schematisch eine Biogaserzeugungsvorrichtung die fünf Bioreaktoren bzw. Faulbehälter 2-1 bis 2-5 gemäß den Figuren 1 bis 4 umfasst. Die fünf Faulbehälter 2-1 bis 2-5 sind über fünf Perkolat-Ableitungen 32-1 bis 32-5 mit einem gemeinsamen Perkolatspeicher 34 verbunden. In jeder Perkolat-Ableitung 32-i ist eine Perkolat-Pumpe 38-i angeordnet, die auch die Funktion des Ventils 36 der Ausführungsform nach den Figuren 1 bis 4 übernimmt. In dem Pumpensumpf der einzelnen Perkolat-Pumpen 38-i ist jeweils ein Drucksensor 40-i zur Ermittlung des Perkolatpegels in dem jeweiligen Faulbehälter 2-i angeordnet. Über eine Perkolat-Rückleiturig 48 mit einer Perkolat-Rückführpumpe 50 kann Perkolat aus dem gemeinsamen Perkolatspeicher 34 in die jeweiligen Faulbehälter 2-i zurückbefördert werden. Über eine Steuereinheit 44 lässt sich der Perkolatpegel 28 steuern und regeln. Die Steuereinheit 44 ist mit den Drucksensoren 40-1 bis 40-5.verbunden und schaltet die Perkolat-Pumpen 38-1 bis 38-5 und die Perkolat-Rückführpumpe 50. Die zugehörigen Steuerleitungen verlaufen entsprechend den Fig. 4 sind jedoch in Fig. 5 aus Gründen der Übersichtlichkeit nicht dargestellt.

Zusätzlich können auch die Faulbehälter 2-i untereinander mit Perkolatleitungen verbunden sein (nicht dargestellt).

### Bezugszeichenliste:

- 1: Bioreaktor
- 2: Faulbehälter
- 4: Biomasse
- 6: Bodenplatte
- 8: Seitenwand
- 9: Seitenwand
- 10: Deckplatte
- 12: Rückwand
- 13: offenen Vorderseite
- 14: Be- und Entladeklappe
- 16: Hydraulik
- 18: Biogasableitung
- 20: Heizeinrichtung
- 22: Sickersaft- bzw. Perkolat-Drainageeinrichtung
- 24: Rinne
- 26: Loch- oder Schlitzblech
- 28: Pegel des Perkolats im Faulbehälter
- 30: Perkolat-Steuereinrichtung
- 32: Perkolat-Ableitung
- 34: Perkolatspeicher
- 36: Ventil
- 38: Perkolat-Pumpe
- 40: Drucksensor 42 Steuerleitungen
- 44: Steuereinheit
- 46: zusätzliche Steuerleitungen

- 2-i: Faulbehälter
- 32-i: Perkolat-Ableitung
- 38-i: Perkolat-Pumpe
- 48: Perkolat-Rückleitung
- 50: Perkolat-Rückführpumpe

## Patentansprüche

1. Bioreaktor zur Methanisierung von Biomasse (4) mit hohem Feststoffanteil, mit
einem gasdicht verschließbaren und flüssigkeitsdicht ausgebildeten Faulbehälter (2),
einer Biogasableitung (18),
einer Be- und Entnahmeöffnung (14) zum Befüllen und entleeren des Faulbehälters mit. Biomasse (4), und
einer Sickersaft- bzw. Perkolat-Drainageeinrichtung (22) im Boden (6) und/oder den Wänden (8, 9, 12) des Faulbehälters (2),
**dadurch gekennzeichnet,**
**dass** die Perkolat-Drainageeinrichtung (22) mit einer Perkolat-Steuereinrichtung (30) verbunden ist, um den Flüssigkeitspegel (28) des Perkolats in dem Faulbehälter (2) zu regeln.

2. Bioreaktor nach Anspruch 1, **dadurch gekennzeichnet, dass** die Perkolat-Steuereinrichtung (30) umfasst:
- einen Perkolatspeicher (34), der über eine Perkolat-Ableitung (32) mit der Perkolat-Drainageeinrichtung (22) verbunden ist,
- ein in der Perkolat-Ableitung (32) angeordnetes Ventil (36), und
- einen Füllstandssensor (40) zur Erfassung des Perkolat-Flüssigkeitspegels (28) in dem Faulbehälter (2).

3. Bioreaktor nach Anspruch 2, **dadurch gekennzeichnet, dass** die Perkolat-Steuereinrichtung (30) eine in der Perkolat-Ableitung (32) angeordnete Perkolat-Pumpe (38) umfasst.

4. Bioreaktor nach Anspruch 2 oder 3, **dadurch gekennzeichnet, dass** der Füllstandssensor eine Drucksensor (40) ist, der in der Perkolat-Ableitung (32) oder im. Faulbehälter (2) angeordnet ist.

5. Bioreaktornach einem der vorhergehenden Ansprüche, **dadurch gekennzeichnet, dass** die Perkolat-Steuereinrichtung (30) eine Perkolat-Rückleitung (48) umfasst, die den Perkolatspeicher (34) mit dem oberen Bereich des Faulbehälters (2) verbindet.

6. Bioreaktor nach einem der vorhergehenden Ansprüche, **dadurch gekennzeichnet, dass** die Be- und Entnahmeöffnung (14) eine vorzugsweise hydraulisch betätigbare Klappe ist, die ebenerdig den Faulbehälter (2) verschließt.

7. Bioreaktor nach Anspruch 6, **gekennzeichnet durch** eine Rückhaltevorrichtung, die in Befüllrichtung hinter der Klappe (14) derart in dem Faulbehälter (2) angeordnet ist, das eingefüllte Biomasse (4) sich wenigstens teilweise an der Rückhaltevorrichtung abstützt.

8. Bioreaktor nach einem der vorhergehenden Ansprüche 2 bis 7, **dadurch gekennzeichnet, dass** in dem Perkolatspeicher (34) Füllkörper eingebracht sind, an denen sich Biogas erzeugende Mikroorganismen anlagern können und dass der Perkolatspeicher (34) eine Biogasentnahmeleitung umfasst.

9. Bioreaktor nach Anspruch 8, **dadurch gekennzeichnet, dass** die Füllkörper im Perkolatspeicher (34) ein Festbett aus Biomasse mit hohem Feststoffanteil ausgebildet sind.

10. Biogaserzeugungsvorrichtung mit einer Mehrzahl von Bioreaktoren (2-i) nach einem der vorhergehenden Ansprüche und einem gemeinsamen Perkolatspeicher (34).

11. Biogaserzeugungsvorrichtung nach Anspruch 10, **dadurch gekennzeichnet, dass** die Mehrzahl der einzelnen Bioreaktoren (2-i) über Perkolat-Verbindungsleitungen untereinander verbunden sind.

## Claims

1. A bioreactor for methanizing biomass (4) having a high solids fraction, comprising
a digestion tank (2) adapted to be sealed in a gas-tight manner and formed to be liquid-tight,
a biogas discharge conduit (18),
a loading and unloading opening (14) for charging and emptying biomass (4) into/from the digestion tank, and
percolating juice or percolate drainage means (22) in the floor (6) and/or walls (8, 9, 12) of the digestion tank (2),
**characterized in that**
the percolate drainage means (22) are connected to percolate control means (30) in order to control the liquid level (28) of the percolate in the digestion tank (2).

2. The bioreactor as claimed in claim 1, **characterized in that** the percolate control means (30) comprise:
- a percolate storage (34) which is connected to the percolate drainage means (22) by way of a percolate discharge conduit (32),
- a valve (36) disposed in the percolate discharge conduit (32), and
- a filling level sensor (40) for detecting the percolate liquid level (28) in the digestion tank (2).

3. The bioreactor as claimed in claim 2, **characterized in that** the percolate control means (30) include a percolate pump (38) disposed in the percolate discharge conduit (32).

4. The bioreactor as claimed in claim 2 or 3, **characterized in that** the filling level sensor is a pressure sensor (40) which is disposed in the percolate discharge conduit (32) or in the digestion tank (2).

5. The bioreactor as claimed in any one of the preceding claims, **characterized in that** the percolate control means (30) include a percolate return line (48) which connects the percolate storage (34) to the upper region of the digestion tank (2).

6. The bioreactor as claimed in any one of the preceding claims, **characterized in that** the loading and unloading opening (14) is a flap, preferably actuated hydraulically, which seals the digestion tank (2) in a manner flush with the ground.

7. The bioreactor as claimed in claim 6, **characterized by** a retaining device which is disposed in the digestion tank (2), in a filling direction behind the flap (14), such that charged biomass (4) is supported at least partially against the retaining device.

8. The bioreactor as claimed in any one of the preceding claims 2 to 7, **characterized in that** filling bodies are incorporated in the percolate storage (34), to which biogas generating microorganisms may attach, and **in that** the percolate storage (34) includes a biogas withdrawal line.

9. The bioreactor as claimed in claim 8, **characterized in that** the filling bodies in the percolate storage (34) form a fixed bed of biomass having a high solids fraction.

10. A biogas generating apparatus comprising a plurality of bioreactors (2-i) as claimed in any one of the preceding claims and a common percolate storage (34).

11. The biogas generating system as claimed in claim 10, **characterized in that** the majority of individual bioreactors (2-i) are interconnected through percolate connection lines.

## Revendications

1. Bioréacteur pour la méthanisation de biomasse (4) à teneur en solides élevée, comprenant
un digesteur (2) pouvant être obturé de manière étanche aux gaz et réalisé de manière étanche aux liquides,
une conduite d'évacuation de biogaz (18),
une ouverture de remplissage et de prélèvement (14) permettant de remplir le digesteur de biomasse (4) et de le vider, et un dispositif de drainage de jus d'écoulement ou percolat (22) dans le fond (6) et/ou dans les parois (8, 9, 12) du digesteur (2),
**caractérisé en ce**
**que** le dispositif de drainage de percolat (22) est relié à un dispositif de commande de percolat (30) afin de régler le niveau de liquide (28) du percolat dans le digesteur (2).

2. Bioréacteur selon la revendication 1, **caractérisé en ce que** le dispositif de commande de percolat (30) comprend :
- un réservoir de percolat (34) qui est relié au dispositif de drainage de percolat (22) par le biais d'une conduite d'évacuation de percolat (32),
- une valve (36) disposée dans la conduite d'évacuation de percolat (32), et
- un capteur de niveau de remplissage (40) pour la détection du niveau de liquide de percolat (28) dans le digesteur (2).

3. Bioréacteur selon la revendication 2, **caractérisé en ce que** le dispositif de commande de percolat (30) comprend une pompe de percolat (38) agencée dans la conduite d'évacuation de percolat (32).

4. Bioréacteur selon la revendication 2 ou 3, **caractérisé en ce que** le capteur de niveau de remplissage est un capteur de pression (40) qui est agencé dans la conduite d'évacuation de percolat (32) ou dans le digesteur (2).

5. Bioréacteur selon l'une quelconque des revendications précédentes, **caractérisé en ce que** le dispositif de commande de percolat (30) comprend une conduite de retour de percolat (48) qui relie le réservoir de percolat (34) à la zone supérieure du digesteur (2).

6. Bioréacteur selon l'une quelconque des revendications précédentes, **caractérisé en ce que** l'ouverture de remplissage et de prélèvement (14) est un volet pouvant être actionné de préférence par voie hydraulique et fermant le digesteur (2) au niveau du sol.

7. Bioréacteur selon la revendication 6, **caractérisé par** un dispositif de retenue qui est agencé dans le digesteur (2) derrière le volet (14) dans le sens de remplissage de telle sorte que la biomasse remplie (4) s'appuie au moins en partie au niveau du dispositif de retenue.

8. Bioréacteur selon l'une quelconque des revendications précédentes 2 à 7, **caractérisé en ce que** des corps de remplissage au niveau desquels peuvent s'accumuler des micro-organismes produisant du biogaz sont intégrés dans le réservoir de percolat (34) et **en ce que** le réservoir de percolat (34) comprend une conduite de prélèvement de biogaz.

9. Bioréacteur selon la revendication 8, **caractérisé en ce que** les corps de remplissage dans le réservoir de percolat (34) forment un lit fixe de biomasse à teneur en solides élevée.

10. Dispositif de production de biogaz avec une pluralité de bioréacteurs (2-i) selon l'une quelconque des revendications précédentes et un réservoir de percolat (34) commun.

11. Dispositif de production de biogaz selon la revendication 10, **caractérisé en ce que** la pluralité des différents bioréacteurs (2-i) sont reliés entre eux par le biais de conduites de liaison de percolat.
